# EUROPEAN PATENT SPECIFICATION

(11) **EP 3 613 372 B1**
(45) Date of publication and mention of the grant of the patent: **08.03.2023**
(21) Application number: 19190532.2
(22) Date of filing: 07.08.2019
(51) Int. Cl.: A61B 18/14, A61B 90/00, A61B 18/00

(54) **CONTACT FORCE SENSOR COMPRISING TUNED AMPLIFIERS**
KONTAKTKRAFTSENSOR MIT ABGESTIMMTEN VERSTÄRKERN
CAPTEUR DE FORCE DE CONTACT COMPRENANT DES AMPLIFICATEURS ACCORDÉS

(30) Priority: 08.08.2018 US 201816058563
(43) Date of publication of application: 26.02.2020
(73) Proprietor: Biosense Webster (Israel) Ltd., Yokneam, 2066717 (IL)
(72) Inventor: GOVARI, Assaf, 2066717 Yokneam (IL)
(74) Representative: Carpmaels & Ransford LLP

(56) References cited:
- EP-A1- 1 502 543
- EP-A1- 2 749 211
- EP-A1- 3 181 083
- WO-A1-2018/071490
- WO-A1-2018/109728
- US-A1- 2017 127 974

## Description

### FIELD OF THE INVENTION

The present invention relates generally to medical probes, and particularly to methods and systems for improving contact force sensing between a probe and tissue.

### BACKGROUND OF THE INVENTION

Some medical probes, such as cardiac ablation catheters, have contact force sensing capabilities.

For example, U.S. Patent Application Publication 2014/0247152 describes a wireless power transmission system that includes, a transmit antenna which in operation produces a wireless field, an amplifier coupled to the transmit antenna, a load sensing circuit coupled to the amplifier and a controller coupled to the load sensing circuit. A monitoring device has one or more sensors and a unique user ID.

U.S. Patent 8,527,046 describes a medical device containing a device for connecting the medical device to a substrate, for furnishing electrical impulses from the medical device to the substrate, for ceasing the furnishing of electrical impulses to the substrate, for receiving pulsed radio frequency fields, for transmitting and receiving optical signals, and for protecting the substrate and the medical device from currents induced by the pulsed radio frequency fields. The medical device contains a control circuit comprised of a parallel resonant frequency circuit.

U.S. Patent Application Publication 2009/0082691 describes a frequency selective monitor that may utilize a heterodyning, chopper-stabilized amplifier architecture to convert a selected frequency band to a baseband for analysis. The frequency selective monitor may be useful in a variety of therapeutic and/or diagnostic applications, such as, a frequency selective signal monitor provided within a medical device or within a sensor coupled to a medical device. The physiological signal may be analyzed in one or more selected frequency bands to trigger delivery of patient therapy and/or recording of diagnostic information.

EP2749211 A1 discloses a catheter that is responsive to external and internal magnetic field generators for generating position data of the catheter position and pressure data to determine pressure exerted on a distal end of the catheter when engaged with tissue. The catheter has a reduced number of sensing coils and reduced number of sensing coil leads for minimizing lead breakage and failure. The catheter includes a distal section adapted for engagement with patient tissue, where the distal section has a proximal portion, a distal portion and a flexible joint with a resilient member adapted to allow axial displacement and angular deflection between the proximal and distal portions of the distal section. The catheter may have three or less sensing coils with three or less leads, each transmitting signals between a respective sensing coil and the signal processor.

WO2018109728 (A1) discloses an electrophysiological catheter system for performing diagnostics and therapies within a cardiac muscle. The system includes a wireless force sensor, mounted to an external surface of a catheter shaft, that detects force exerted on a catheter tip and wirelessly transmits a signal indicative of the sensed force to a wireless transceiver in proximity thereto.

### SUMMARY OF THE INVENTION

The present invention provides a medical probe having a contact sensor as claimed in claim 1.

In some embodiments, the medical probe includes a processor, which is configured to receive the signals amplified by the one or more narrow-band amplifiers, and to estimate, based on the received signals, a deflection of the first end relative to a longitudinal axis of the elastic element at the second end. In other embodiments, the signals include radio-frequency (RF) signals. In yet other embodiments, the transmitter includes a dipole radiator.

In an embodiment, the transmitter includes one or more coils. In another embodiment, the one or more receiving antennas include one or more respective coils. In yet another embodiment, each of the narrow-band amplifiers includes a respective field effect transistor (FET).

In some embodiments, each of the narrow-band amplifiers includes a respective resonant circuit, which is coupled to the FET and has a resonant frequency that matches the given range of frequencies. In other embodiments, each of the narrow-band amplifiers includes a respective resonant circuit having a range of resonance frequencies that matches the given range of frequencies.

There is additionally provided, in accordance with the present invention, a method for producing a medical probe having a contact force sensor as claimed in claim 3.

There is further provided, in accordance with the present invention, a method of operating a contact force sensor in a medical probe as claimed in claim 12.

In some embodiments, the method includes receiving the signals amplified by the one or more narrow-band amplifiers, and estimating, based on the received signals, a deflection of the first end relative to a longitudinal axis of the elastic element at the second end.

The present invention will be more fully understood from the following detailed description of the embodiments thereof, taken together with the drawings in which:

### BRIEF DESCRIPTION OF THE DRAWINGS

Fig. 1 is a schematic, pictorial illustration of a catheterization system, in accordance with an embodiment of the present invention; and
Fig. 2 is a schematic, pictorial illustration of a catheter distal end comprising a contact force sensor, in accordance with an embodiment of the present invention.

### DETAILED DESCRIPTION OF EMBODIMENTS

### OVERVIEW

Some medical procedures, such as radio-frequency (RF) ablation in heart tissue, require good physical contact between an ablation electrode of a catheter and the tissue.

The present invention provides improved techniques for sensing contact force between a catheter distal-end and heart tissue of a patient. In some embodiments, the catheter comprises a distal-end assembly comprising one or more RF ablation electrodes, and a contact force sensor, configured to sense the contact force applied between the distal-end assembly and the heart tissue.

In some embodiments, the contact force sensor comprises a spring or other elastic element, having first and second ends. The spring is mounted along the catheter longitudinal axis, such that the first end faces the catheter distal-end and the second end faces the catheter proximal-end.

The contact force sensor comprises a transmitter, which is coupled to the first end of the spring, and is configured to transmit signals at a predefined frequency. The contact force sensor may further comprise multiple (e.g., nine) receiving antennas, which are coupled to the second end of the spring, and are configured to receive the transmitted signals. In some embodiments, each antenna comprises a coil, which is electrically coupled to a respective narrow band amplifier having a resonant circuit that matches the predefined frequency of the transmitted signals.

When an operator brings the distal-end assembly into physical contact with the heart tissue, each coil produces an electrical signal, referred to herein as a "force signal." The narrow band amplifier increases the signal-to-noise ratio (SNR) of the force signal, which is transmitted via additional devices of the catheter, to a processor. In principle, it is possible to amplify the force signals using a wideband amplifier, however, wideband amplifiers are configured to amplify signals in a broad range of frequencies, including noise signals that interfere with the force signals and reducing their SNR. Therefore, it is important to increase the SNR of the force signals, so as to improve the sensitivity of the contact force calculation.

In some embodiments, the processor is configured to calculate the contact force applied between the distal-end assembly and the tissue by comparing between the force signals received from all nine coils. The processor is further configured to display the estimated contact force to the user.

The disclosed techniques improve the quality and accuracy of various procedures, such as ablation and electro-potential (EP) mapping, by improving the sensitivity of the contact force sensing between the catheter and the tissue in question.

### SYSTEM DESCRIPTION

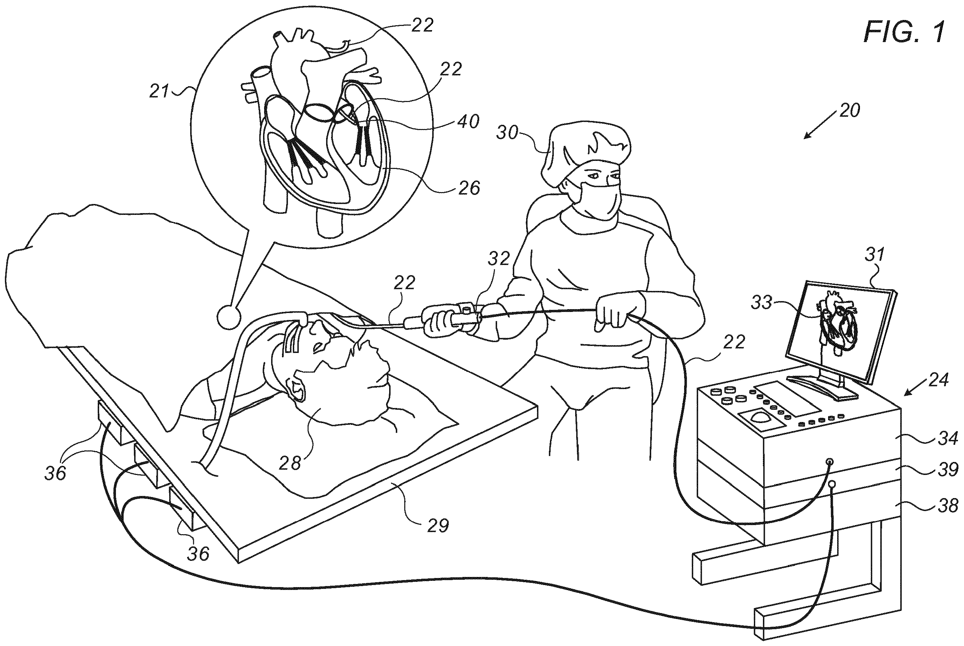

Fig. 1 is a schematic, pictorial illustration of a catheterization system 20, in accordance with an embodiment of the present invention. System 20 comprises a probe, in the present example a cardiac catheter 22, and a control console 24. In the embodiment described herein, catheter 22 may be used for any suitable therapeutic and/or diagnostic purposes, such as for ablating tissue in a patient heart 26.

Console 24 comprises a processor 34, typically a general-purpose computer, with suitable front end and interface circuits for receiving signals from catheter 22 and for controlling the other components of system 20 described herein. Processor 34 may be programmed in software to carry out the functions that are used by the system, and the processor stores data for the software in a memory 38. The software may be downloaded to console 24 in electronic form, over a network, for example, or it may be provided on non-transitory tangible media, such as optical, magnetic or electronic memory media. Alternatively, some or all of the functions of processor 34 may be carried out by dedicated or programmable digital hardware components.

An operator 30 (such as an interventional cardiologist) inserts catheter 22 through the vascular system of a patient 28 lying on a table 29. Catheter 22 comprises an insertion tube, and a distal-end assembly 40 that comprises one or more position sensors (not shown.) Operator 30 moves assembly 40 of catheter 22 in the vicinity of the target region in heart 26 by manipulating catheter 22 with a manipulator 32 near the proximal end of the catheter as shown in an inset 21. The proximal end of catheter 22 is connected to interface circuitry in processor 34.

In some embodiments, system 20 comprises a magnetic position tracking system configured to track the position of distal-end assembly 40 in the body of patient 28. The position of distal-end assembly 40 in the heart cavity is typically measured using one or more magnetic position sensors of the magnetic position tracking system. In the example of Fig. 1, console 24 comprises a driver circuit 39, which drives magnetic field generators 36 placed at known positions external to patient 28 lying on table 29, e.g., below the patient's torso.

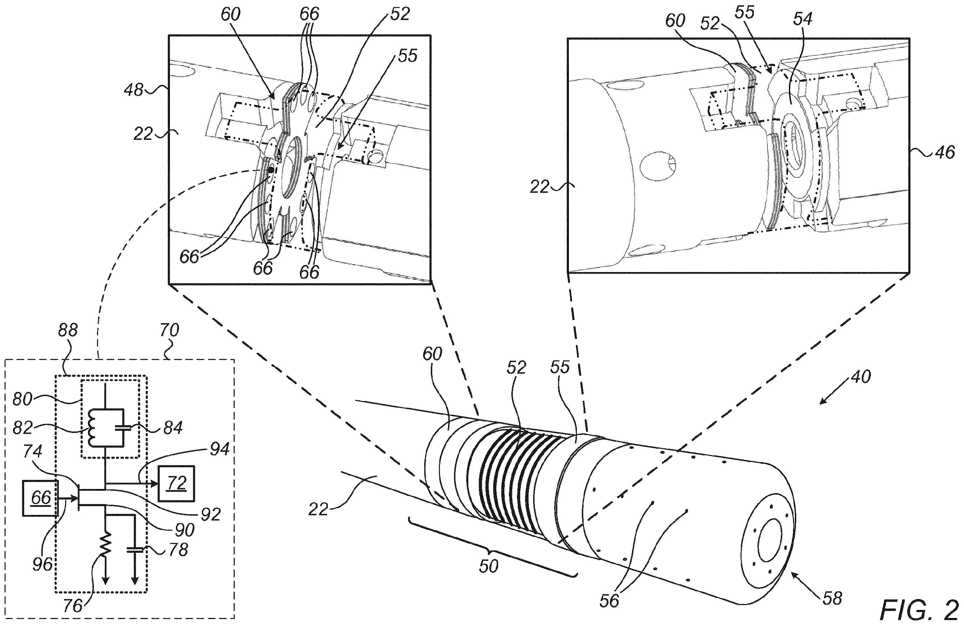

Distal-end assembly 40 typically comprises one or more position sensors and other devices coupled thereto, such as a contact force sensor and ablation electrodes (both shown in Fig. 2 below). After operator 30 navigates catheter 22 to an ablation site, the distal-end assembly is brought into contact with tissue in the inner surface of heart 26. In some embodiments, the contact force sensor is configured to produce an electrical signal indicative of the contact force between distal-end assembly 40 and the tissue of heart 26. When distal-end assembly 40 is positioned at the ablation site and having appropriate contact force to the tissue, operator 30 applies radio-frequency (RF) power for ablating the tissue.

The various sensors and electrodes of assembly 40 are connected to interface circuitry in processor 34 at the catheter proximal end. Operator 30 can view the position of assembly 40 in an image 33 of heart 26 on a user display 31.

This method of position sensing is implemented in magnetic position tracking systems, for example in the CARTO^{™} system, produced by Biosense Webster Inc. (Irvine, Calif.) and is described in detail in U.S. Patents 5, 391, 199, 6, 690, 963, 6, 484, 118, 6, 239, 724, 6, 618, 612 and 6,332,089, in PCT Patent Publication WO 96/05768, and in U.S. Patent Application Publications 2002/0065455 A1, 2003/0120150 A1 and 2004/0068178 A1.

This particular configuration of catheter 22 is shown by way of example, in order to illustrate certain problems that are addressed by embodiments of the present invention and to demonstrate the application of these embodiments in enhancing the performance of such a catheter. Embodiments of the present invention, however, are by no means limited to this specific sort of example medical probe, and the principles described herein may similarly be applied to other sorts of catheterization and position tracking systems.

### CONTACT FORCE SENSING BETWEEN THE DISTAL-END ASSEMBLY AND HEART TISSUE

Fig. 2 is a schematic, pictorial illustration of a distal-end assembly 40, in accordance with an embodiment of the present invention. In some embodiments, distal-end assembly 40 comprises an ablation electrode, which is coupled to a tip 58 of distal-end assembly 40, and is configured to transmit RF power for ablating tissue of heart 26.

In some embodiments, distal-end assembly 40 is configured to irrigate, through irrigation holes 56, the tissue area during the RF ablation. As describes in Fig. 1 above, distal-end assembly 40 comprises a force sensor 50, configured to sense the level of contact force applied between distal-end assembly 40 and the tissue of heart 26.

In some embodiments, force sensor 50 comprises an elastic element, such as a double helix spring 52. In some embodiments, a transmitter (Tx) 55 and a receiver (Rx) 60, are coupled, respectively, to the distal and proximal ends of spring 52.

Reference is now made to an inset 46, which is a schematic pictorial illustration of Tx 55 of force sensor 50. Note that, for the sake of clarity, only the edges of spring 52 are illustrated in dashed lines, so as to show the inner structure of Tx 55. As depicted above and shown by the dashed lines, spring 52 is coupled between Tx 55 and Rx 60.

In some embodiments, Tx 55 comprises a dipole radiator or a coil 54, which is configured to transmit signals, and is typically printed on a suitable substrate, such as a flexible printed circuit board (PCB).

In some embodiments, Tx 55 is configured to transmit signals at a selected frequency, or at a given range of frequencies, such as a range of RF frequencies (e.g., 1 KHz - 100 MHz).

Reference is now made to an inset 48, which is a schematic pictorial illustration of Rx 60 of force sensor 50. Note that force sensor 50 is shown from different perspectives in insets 46 and 48 so as to depict Tx 55 and Rx 60 in detail in insets 46 and 48, respectively.

In some embodiments, Rx 60 comprises one or more antennas, in the present example nine antennas made from nine respective coils 66. In the example of inset 48, coils 66 are arranged in three sections of Rx 60, each section comprising three coils 66. Note that Rx 60 may comprise any other suitable number of coils 66 arrangement in any suitable configuration.

In some embodiments, each coil 66 is configured to receive the RF signals transmitted by coil 54 of Tx 55. In response to receiving the RF signals, coil 66 is further configured to produce electrical signals, also referred to herein as "force signals," indicative of the relative position and orientation of the respective coil 66 relative to Tx 55.

In some embodiments, the produced force signals are subsequently processed (e.g., amplified and filtered), as will be described in detail below, and are transmitted to a processing unit of system 20, such as processor 34. In some embodiments, processor 34 is configured to receive the force signals from some or all coils 66. Processor 34 is further configured, based on the magnitudes of the nine force signals received from the respective coils 66, to estimate the deflection of the first end of spring 52 (e.g., at Tx 55) relative to a longitudinal axis of catheter 22 at the second end of spring 52 (e.g., at Rx 60).

In these embodiments, processor 34 is configured to estimate, based on a comparison among the signals received from all coils 66, the force applied on spring 52. In alternative embodiments, force sensor 50 may have a different configuration, for example Rx 60 may comprise a single coil 66. In these embodiments, processor 34 is configured to estimate the force applied on spring 52 based on the force signals received from the single coil.

In the example embodiment of Fig. 2, coils 66 are arranged on the same plane of Rx 60. In another embodiment, coils 66 may be arranged in any other suitable configuration.

In some embodiments, coils 66 are further configured to sense position signals transmitted, for example, by field generators 36. The sensed position signals are indicative of the position of distal-end assembly 40 in the coordinate system of the position tracking system described in Fig. 1 above. Tx 55 and field generators 36 typically transmit signals at different frequencies, so as to prevent interference therebetween.

In some embodiments, processor 34 is configured to receive the position signals of coils 66, and to estimate, based on the received signals, the position of distal-end assembly 40, e.g., in the coordinate system of the magnetic position tracking system.

### IMPROVING CONTACT FORCE SENSING ACCURACY USING TUNED AMPLIFIERS

Reference is now made to an inset 70. In some embodiments, the force signals sensed by each coil 66 may be amplified using a respective wideband amplifier 72. In an embodiment, the nine amplified signals are filtered and sent to processor 34. Wideband amplifiers are configured to amplify signals in a broad range of frequencies, including noise signals that interfere with the force signals, and therefore it is important to provide wideband amplifiers 72 with high signal-to-noise ratio (SNR) input force signals.

In some embodiments, each coil 66 is electrically coupled, via one or more lines 96, to a respective narrow band amplifier 88, which is configured to amplify a selected narrow band of frequencies, received from coil 66. Note that narrow band amplifier 88 is duplicated per coil 66. In other words, Rx 60 comprises nine narrow band amplifiers 88. In some embodiments, narrow band amplifier 88 comprises a low-noise field-effect transistor (FET) 74 having a noise level lower than 2nV/VHz, or any other suitable noise level. In some embodiments, a source 90 of FET 74 is electrically coupled to a grounded resistor 76 and, in parallel, to a grounded capacitor 78.

In some embodiments, a tuned circuit, referred to herein as a resonant circuit 80, is electrically coupled to a drain 92 of FET 74. In an embodiment, resonant circuit 80 is a parallel circuit comprising an inductive coil 82 and a capacitor 84 electrically coupled in parallel. In some embodiments, resonant circuit 80 is tuned (e.g., having a resonance frequency that matched) to the frequency of the signals transmitted from Tx 55.

As described above, Tx 55 is further configured to transmit signals at a given range of frequencies. In these embodiments, resonant circuit 80 is tuned to the given range of frequencies.

In some embodiments, the quality factor (Q) of each inductive coil 82 is relatively low, e.g., 10 or even lower. Nevertheless, the low Q of coil 82 is compensated for by the high gain factor, also known as "beta," of amplifier 88, which increases the effective value of Q.

In some embodiments, the force signal produced by each coil 66 is amplified by narrow band amplifier 88. The amplified signal has substantially higher SNR compared to the raw force signal produced by coil 66.

In some embodiments, the high SNR force signal received from each coil 66 and amplified by the respective narrow band amplifier 88, is transmitted via one or more lines 94, to wideband amplifier 72 for further amplification. Subsequently, each force signal received from respective amplifier 88 may be further processed (e.g., filtered) and transmitted to processor 34, or to any other suitable processing unit of system 20.

In some embodiments, each force signal received from respective one or more lines 94 may be processed individually as described above. In other embodiments, all the force signals received from the respective nine narrow band amplifiers 88 may be grouped (e.g., summed up) at any suitable stage between amplifiers 88 and processor 34 so that filtering, for example, is carried out on the grouped force signals.

The configuration shown in Fig. 2 is depicted purely by way of example. In alternative embodiments, force sensor 50 may comprise any other suitable type of elastic element, instead of, or in addition to the double helix spring described above. In other embodiments, Rx 60 may comprise any suitable number of antennas made from coils or using any other suitable techniques. Furthermore, embodiments of the present invention are by no means limited to this specific sort of exemplary narrow band amplifier, and the principles described herein may similarly be applied to other sorts of amplifiers applied in various sorts of force sensors or other medical devices and systems.

Although the embodiments described herein mainly address contact force sensors in cardiac ablation procedures, the methods and systems described herein can also be used in other applications, such as in image guided surgery.

It will thus be appreciated that the embodiments described above are cited by way of example, and that the present invention is not limited to what has been particularly shown and described hereinabove. Rather, the scope of the present invention is defined by the claims and, as far as they fall under the scope of the independent claims, includes both combinations and sub-combinations of the various features described hereinabove.

## Claims

1. A medical probe (22) having a contact force sensor (50), the contact force sensor (50) comprising:
an elastic element (52) having first and second ends;
a transmitter (55), coupled to the first end and configured to transmit signals in a given range of frequencies;
one or more receiving antennas (60) coupled to the second end and configured to receive the signals; and
one or more narrow-band amplifiers (88), which have a pass-band that matches the given range of frequencies and which are configured to amplify the signals received by the one or more receiving antennas, respectively.

2. The medical probe (22) according to claim 1, and comprising a processor (34), which is configured to receive the signals amplified by the one or more narrow-band amplifiers (88), and to estimate, based on the received signals, a deflection of the first end relative to a longitudinal axis of the elastic element (52) at the second end.

3. A method for producing a medical probe (22) having a contact force sensor (50), the method comprising:
providing an elastic element (52) having first and second ends;
coupling, to the first end, a transmitter (55) for transmitting signals in a given range of frequencies;
coupling, to the second end, one or more receiving antennas (60) for receiving the signals; and
coupling, to the receiving antennas one or more respective narrow-band amplifiers (88), which have a pass-band that matches the given range of frequencies for amplifying the signals received by the one or more receiving antennas, respectively.

4. The method according to claim 3, and comprising coupling the one or more receiving antennas (60) to a processor (34) for receiving the signals amplified by the one or more narrow-band amplifiers (88), and for estimating, based on the received signals, a deflection of the first end relative to a longitudinal axis of the elastic element (52) at the second end.

5. The medical probe (22) according to claim 1 or the method according to claim 3, wherein the signals comprise radio-frequency (RF) signals.

6. The medical probe (22) according to claim 1 or the method according to claim 3, wherein the transmitter comprises a dipole radiator.

7. The medical probe (22) according to claim 1 or the method according to claim 3, wherein the transmitter comprises one or more coils (54).

8. The medical probe (22) according to claim 1 or the method according to claim 3, wherein the one or more receiving antennas comprise one or more respective coils (66) .

9. The medical probe (22) according to claim 1 or the method according to claim 3, wherein each of the narrow-band amplifiers comprises a respective field effect transistor, FET, (74).

10. The medical probe (22) or the method according to claim 9, wherein each of the narrow-band amplifiers (88) comprises a respective resonant circuit (80), which is coupled to the FET (74) and has a resonant frequency that matches the given range of frequencies.

11. The medical probe (22) according to claim 1 or the method according to claim 3, wherein each of the narrow-band amplifiers (88) comprises a respective resonant circuit (80) having a range of resonance frequencies that matches the given range of frequencies.

12. A method of operating a contact force sensor in a medical probe, comprising:
transmitting signals in a given range of frequencies, using a transmitter (55) coupled to a first end of an elastic element (52), which is disposed in the medical probe (22) and has first and second ends;
receiving the signals, using one or more receiving antennas (60) coupled to the second end; and
amplifying the signals received by the one or more receiving antennas, using one or more respective narrow-band amplifiers (88), which have a pass-band that matches the given range of frequencies.

13. The method according to claim 12, and comprising receiving the signals amplified by the one or more narrow-band amplifiers (88), and estimating, based on the received signals, a deflection of the first end relative to a longitudinal axis of the elastic element (52) at the second end.

## Patentansprüche

1. Medizinische Sonde (22) mit einem Kontaktkraftsensor (50), wobei der Kontaktkraftsensor (50) umfasst:
ein elastisches Element (52) mit einem ersten und einem zweiten Ende;
einen Sender (55), der mit dem ersten Ende gekoppelt und dafür ausgelegt ist, Signale in einem bestimmten Frequenzbereich zu übertragen;
eine oder mehrere Empfangsantennen (60), die mit dem zweiten Ende gekoppelt und dafür ausgelegt sind, die Signale zu empfangen; und
einen oder mehrere Schmalbandverstärker (88), die ein Durchlassband aufweisen, das mit dem vorgegebenen Frequenzbereich übereinstimmt, und die dafür ausgelegt sind, die von den ein oder mehreren Empfangsantennen empfangenen Signale zu verstärken.

2. Medizinische Sonde (22) gemäß Anspruch 1 und einen Prozessor (34) umfassend, die dafür ausgelegt ist, die von den ein oder mehreren Schmalbandverstärkern (88) verstärkten Signale zu empfangen und basierend auf den empfangenen Signalen eine Auslenkung des ersten Endes relativ zu einer Längsachse des elastischen Elements (52) am zweiten Ende zu schätzen.

3. Verfahren zur Herstellung einer medizinischen Sonde (22) mit einem Kontaktkraftsensor (50), wobei das Verfahren umfasst:
Bereitstellen eines elastischen Elements (52) mit einem ersten und einem zweiten Ende;
Koppeln, mit dem ersten Ende, eines Senders (55) zum Übertragen von Signalen in einem bestimmten Frequenzbereich;
Koppeln, mit dem zweiten Ende, einer oder mehrerer Empfangsantennen (60) zum Empfangen der Signale; und
Koppeln, mit den Empfangsantennen, von einem oder mehreren Schmalbandverstärkern (88), die ein Durchlassband aufweisen, das mit dem vorgegebenen Frequenzbereich übereinstimmt, um die von den ein bzw. mehreren Empfangsantennen empfangenen Signale zu verstärken.

4. Verfahren gemäß Anspruch 3 und umfassend, die ein oder mehreren Empfangsantennen (60) mit einem Prozessor (34) zu koppeln, um die von den ein oder mehreren Schmalbandverstärkern (88) verstärkten Signale zu empfangen und basierend auf den empfangenen Signalen eine Auslenkung des ersten Endes relativ zu einer Längsachse des elastischen Elements (52) am zweiten Ende schätzen.

5. Medizinische Sonde (22) gemäß Anspruch 1 oder Verfahren gemäß Anspruch 3, wobei die Signale Hochfrequenz (HF) -Signale umfassen.

6. Medizinische Sonde (22) gemäß Anspruch 1 oder Verfahren gemäß Anspruch 3, wobei der Sender einen Dipolstrahler umfasst.

7. Medizinische Sonde (22) gemäß Anspruch 1 oder Verfahren gemäß Anspruch 3, wobei der Sender eine oder mehrere Spulen (54) umfasst.

8. Medizinische Sonde (22) gemäß Anspruch 1 oder Verfahren gemäß Anspruch 3, wobei die ein oder mehreren Empfangsantennen eine oder mehrere entsprechende Spulen (66) umfassen.

9. Medizinische Sonde (22) gemäß Anspruch 1 oder Verfahren gemäß Anspruch 3, wobei jeder der Schmalbandverstärker einen entsprechenden Feldeffekttransistor, FET, (74) umfasst.

10. Medizinische Sonde (22) oder Verfahren gemäß Anspruch 9, wobei jeder der Schmalbandverstärker (88) einen entsprechenden Resonanzkreis (80) umfasst, der mit dem FET (74) gekoppelt ist und eine Resonanzfrequenz aufweist, die mit dem gegebenen Frequenzbereich übereinstimmt.

11. Medizinische Sonde (22) gemäß Anspruch 1 oder Verfahren gemäß Anspruch 3, wobei jeder der Schmalbandverstärker (88) einen entsprechenden Resonanzkreis (80) umfasst, der einen Resonanzfrequenzbereich aufweist, der mit dem gegebenen Frequenzbereich übereinstimmt.

12. Verfahren zum Betreiben eines Kontaktkraftsensors in einer medizinischen Sonde,
umfassend:
Übertragen von Signalen in einem bestimmten Frequenzbereich mit Hilfe eines Senders (55), der mit einem ersten Ende eines elastischen Elements (52) gekoppelt ist, das in der medizinischen Sonde (22) angeordnet ist und ein erstes und ein zweites Ende hat;
Empfangen der Signale unter Verwendung einer oder mehrerer Empfangsantennen (60), die mit dem zweiten Ende gekoppelt sind; und
Verstärken der von den ein oder mehreren Empfangsantennen empfangenen Signale mit Hilfe eines oder mehrerer entsprechender Schmalbandverstärker (88), die ein Durchlassband aufweisen, das mit dem gegebenen Frequenzbereich übereinstimmt.

13. Verfahren gemäß Anspruch 12 und umfassend, die von den ein oder mehreren Schmalbandverstärkern (88) verstärkten Signale zu empfangen und basierend auf den empfangenen Signalen eine Auslenkung des ersten Endes relativ zu einer Längsachse des elastischen Elements (52) am zweiten Ende zu schätzen.

## Revendications

1. Sonde médicale (22) présentant un capteur de force de contact (50), le capteur de force de contact (50) comprenant :
un élément élastique (52) présentant des première et seconde extrémités ;
un émetteur (55), couplé à la première extrémité et configuré pour émettre des signaux dans une plage de fréquences donnée ;
une ou plusieurs antennes de réception (60) couplées à la seconde extrémité et configurées pour recevoir les signaux ; et
un ou plusieurs amplificateurs à bande étroite (88), qui ont une bande passante qui correspond à la plage de fréquences donnée et qui sont configurés pour amplifier les signaux reçus par la ou les antennes de réception, respectivement.

2. Sonde médicale (22) selon la revendication 1, et comprenant un processeur (34), qui est configuré pour recevoir les signaux amplifiés par le ou les amplificateurs à bande étroite (88), et pour estimer, sur la base des signaux reçus, une déviation de la première extrémité par rapport à un axe longitudinal de l'élément élastique (52) au niveau de la seconde extrémité.

3. Procédé permettant de fabriquer une sonde médicale (22) présentant un capteur de force de contact (50), le procédé comprenant :
la fourniture d'un élément élastique (52) présentant des première et seconde extrémités ;
le couplage, à la première extrémité, d'un émetteur (55) pour émettre des signaux dans une plage de fréquences donnée ;
le couplage, à la seconde extrémité, d'une ou de plusieurs antennes de réception (60) pour recevoir les signaux ; et
le couplage, aux antennes de réception, d'un ou de plusieurs amplificateurs à bande étroite (88) respectifs, qui ont une bande passante qui correspond à la plage de fréquences donnée pour amplifier les signaux reçus par la ou les antennes de réception, respectivement.

4. Procédé selon la revendication 3, et comprenant le couplage de la ou des antennes de réception (60) à un processeur (34) pour recevoir les signaux amplifiés par le ou les amplificateurs à bande étroite (88), et pour estimer, sur la base des signaux reçus, une déviation de la première extrémité par rapport à un axe longitudinal de l'élément élastique (52) au niveau de la seconde extrémité.

5. Sonde médicale (22) selon la revendication 1 ou procédé selon la revendication 3, dans lesquels les signaux comprennent des signaux radiofréquence (RF).

6. Sonde médicale (22) selon la revendication 1 ou procédé selon la revendication 3, dans lesquels l'émetteur comprend un radiateur dipôle.

7. Sonde médicale (22) selon la revendication 1 ou procédé selon la revendication 3, dans lesquels l'émetteur comprend une ou plusieurs bobines (54).

8. Sonde médicale (22) selon la revendication 1 ou procédé selon la revendication 3, dans lesquels la ou les antennes de réception comprennent une ou plusieurs bobines (66) respectives.

9. Sonde médicale (22) selon la revendication 1 ou procédé selon la revendication 3, dans lesquels chacun des amplificateurs à bande étroite comprend un transistor à effet de champ, TEC, (74) respectif.

10. Sonde médicale (22) ou procédé selon la revendication 9, dans lesquels chacun des amplificateurs à bande étroite (88) comprend un circuit résonnant (80) respectif, qui est couplé au TEC (74) et a une fréquence de résonance qui correspond à la plage de fréquences donnée.

11. Sonde médicale (22) selon la revendication 1 ou procédé selon la revendication 3, dans lesquels chacun des amplificateurs à bande étroite (88) comprend un circuit résonnant (80) respectif ayant une plage de fréquences de résonance qui correspond à la plage de fréquences donnée.

12. Procédé d'utilisation d'un capteur de force de contact dans une sonde médicale, comprenant :
l'émission de signaux dans une plage de fréquences donnée, au moyen d'un émetteur (55) couplé à une première extrémité d'un élément élastique (52), qui est disposé dans la sonde médicale (22) et qui présente des première et seconde extrémités ;
la réception des signaux, au moyen d'une ou de plusieurs antennes de réception (60) couplées à la seconde extrémité ; et
l'amplification des signaux reçus par la ou les antennes de réception, au moyen d'un ou de plusieurs amplificateurs à bande étroite (88) respectifs, qui ont une bande passante qui correspond à la plage de fréquences donnée.

13. Procédé selon la revendication 12, et comprenant la réception des signaux amplifiés par le ou les amplificateurs à bande étroite (88), et l'estimation, sur la base des signaux reçus, d'une déviation de la première extrémité par rapport à un axe longitudinal de l'élément élastique (52) au niveau de la seconde extrémité.
